# EUROPEAN PATENT SPECIFICATION

(11) **EP 1 073 716 B1**
(45) Date of publication and mention of the grant of the patent: **28.04.2004**
(21) Application number: 99919396.4
(22) Date of filing: 28.04.1999
(51) Int. Cl.: C12N 5/00, B01F 17/00, B03D 1/016, C07K 16/28, G01N 33/48

(54) **IMPROVEMENTS IN OR RELATING TO SEPARATION PROCESSES**
VERBESSERUNG VON TRENNUNGSVERFAHREN
PROCEDES DE SEPARATION AMELIORES

(30) Priority: 28.04.1998 GB 9809083; 28.04.1998 GB 9809085
(43) Date of publication of application: 07.02.2001
(73) Proprietor: Amersham Health AS, 0401 Oslo (NO)
(72) Inventor: CUTHBERTSON, Alan,Amersham Health AS, N-0401 Oslo (NO); RONGVED, Pal, N-1450 Nesoddtangen (NO); L VHAUG, Dagfinn,Amersham Health AS, N-0401 Oslo (NO); FJERDINGSTAD, Hege,Amersham Health AS, N-0401 Oslo (NO); SOLBAKKEN, Magne,Amersham Health AS, N-0401 Oslo (NO); GODAL, Aslak, N-0365 Oslo (NO)
(74) Representative: Hammett, Audrey Grace Campbell
(86) International application number: PCT/GB1999/001317
(87) International publication number: WO 1999/055837

(56) References cited:
- WO-A-97/29783
- WO-A-98/18500
- WO-A-99/13918
- DE-A- 2 642 944
- US-A- 4 276 885
- US-A- 4 620 980
- US-A- 4 971 731
- US-A- 5 116 724
- VILLANUEVA ET AL: "Microbubbles targeted to intercellular adhesion molecule-1 bind to activated coronary artery endothelial cells" CIRCULATION,US,DALLAS, TX, vol. 98, no. 1, page 1-5 XP002092048 ISSN: 0009-7322

## Description

This invention relates the separation of components from samples, more particularly to separation methods employing floatable gas microbubbles, and to apparatus useful in such methods.

The separation of target components from samples is a problem faced daily by workers in a wide variety of fields, including water purification, industrial chemistry, medicine and biotechnology. The need to separate particular components arises because samples are frequently heterogeneous and a specific component may need to be recovered or removed from such a sample before further manipulation or usage of the sample or component occurs. Isolation and purification of components of the order of microns in size usually includes a filtration or centrifugation step to remove certain components, followed by a step concentrating the component of interest; a chromatographic step may also be included. Thereafter more refined purification steps are often needed to obtain the desired component in sufficient purity. It would clearly be advantageous if components could be separated in one simple step without the use of such processes, which may often be expensive and cumbersome.

Comparatively recently, separation techniques mediated by antibody-antigen reactions have been employed in both research and clinical laboratories, largely due to the advent of monoclonal antibodies and their particular specificity for defined antigen epitopes. Thus, in fields such as biotechnology, an alternative to time consuming filtration/centrifugation and concentration techniques involves the use of targeted antibodies to bind to particular components of interest.

One technique which utilises such antibody-antigen binding is the separation of, for example, blood components or cells using superparamagnetic polymer particles such as Dynabeads®. Superparamagnetic particles exhibit magnetic properties only in the presence of a magnetic field. Their use in separation procedures has been known for a number of years and is now widespread.

Superparamagnetic polymer particles may be coated with a specific ligand and added to a heterogeneous target suspension to bind a desired target. The resulting target/superparamagnetic particle complex may be fixed simply by using a magnet and the target-free suspension may then be withdrawn from the system. Alternatively, a primary antibody having specificity for the target may first be added to the suspension and, after removal of any excess antibody, magnetic polymer particles carrying a secondary antibody having specificity for the primary antibody may be added to bind the target via the primary antibody. Magnetic separation may then be effected as above.

Magnetic separation of cells does, however, suffer from a number of disadvantages. Firstly, when a magnetic field is applied to a sample, superparamagnetic particles and target/particle complexes will rapidly be drawn through the sample towards the magnet. Since the polymer particles are hard and may typically be of similar size to cells, this rapid movement can cause significant damage to target cells in the sample.

Moreover, detachment of separated components from the polymer particles can be a difficult and time-consuming process. A representative technique involves use of a polyclonal antibody that reacts with Fab-fragments of monoclonal antibodies to effect direct dissociation of the antigen-antibody binding. This technique is only suitable for use with certain types of polymer particle and certain monoclonal antibodies. Alternative detachment methods include overnight incubation at 37°C, enzymatic cleavage and the introduction of reagents which compete for the same target as the polymer particles.

Due to the complicated nature of these detachment techniques, magnetic polymer particles are predominantly employed in "negative" component separations, i.e. processes in which unwanted components are bound to the magnetic particles and isolated, leaving the desired components in the sample. Their use in "positive" selections, where a specific component is targeted and isolated, is comparatively limited.

As an alternative to magnetic separation procedures such as those discussed above, a number of separation processes using floatable beads or low density particles have been proposed. In Transplantation ***46(4),*** pp. 558-563, [1988] a technique is described for removing leukemia or lymphoma cells from autologous bone marrow. The method utilises low density polypropylene beads coated with a monoclonal antibody. The target cells bind to the monoclonal antibodies attached to the polypropylene beads and float to the surface of the suspension where they may be removed by decantation, leaving normal marrow cells remaining in the suspension.

In US-A-5116724 a product for separation of cells and viruses is described. It consists of particles which are 'floatable', i.e. which have a lower density than the medium in which they are used. Suitable particles, which may be coated with macromolecules capable of specific fixation to target cells, are said to include particles of different shapes and dimensions, comprising materials such as low density polyethylene or polypropylene.

DE-A-2642944 describes the recovery of bacterial cell masses from aqueous culture solution by flotation using electrolytically generated gas bubbles.

It will be appreciated that techniques using floatable beads etc. will inevitably suffer the same limitations as those using magnetic particles insofar as positive component separations are concerned. Also, the relatively low density difference between typical low density polymer particles and aqueous culture media may limit the effectiveness of such flotation separations.

Thus, there remains a need for separation techniques which ensure that target components remain undamaged during separation procedures, which are useful for both negative and positive selection of components and which permit efficient and highly selective separation of target components.

The present invention is based on the finding that highly efficient component separation may be achieved using flotation methods in which target component becomes bound to encapsulated gas microbubbles. The efficiency of such separations is enhanced by the substantial density difference between gas microbubbles and liquid sample media, so that the process is capable of high sensitivity. Flotation separations inherently proceed more gently than magnetic separations and the gas microbubbles may advantageously be prepared using flexible encapsulating materials, so that the possibility of causing damage to sensitive target components such as cells during separation may thus be minimised. The use of encapsulated gas microbubbles also permits ready removal of the microbubbles from the target component after separation, simply by bursting the microbubbles.

Such separation procedures differ from existing flotation separation techniques, for example such as are used in the separation of minerals or the purification of oil-contaminated water, in that currently known flotation separations use free gas bubbles or microbubbles generated in situ rather than pre-prepared encapsulated gas microbubbles. It will be appreciated that it is not possible to use free gas bubbles/microbubbles to perform procedures such as the affinity separation of cells.

According to one aspect of the present invention there is provided a process for the separation of target material from a liquid sample which comprises coupling the target to targetable encapsulated gas microbubbles, allowing the microbubbles and coupled target to float to the surface of the sample to form a floating microbubble/target layer, separating this layer from the sample, and either removing the microbubbles from the target (in the case of a positive selection) or recovering target-free sample material (in the case of a negative selection).

Encapsulated microbubbles which may be useful in accordance with the invention include any stabilised microbubbles which may be prepared in a targetable form. Depending on the intended application of the microbubbles the encapsulating material and the gas content may be biocompatible or non-biocompatible; the former will naturally be preferred in separations involving cells, biomolecules etc. Representative examples of microbubbles include those which are suitable for use in targetable contrast agent formulations, especially targetable ultrasound contrast agent formulations, and include microbubbles of gas stabilised (e.g. at least partially encapsulated) by a coalescence-resistant surface membrane (for example gelatin, e.g. as described in WO-A-8002365), a filmogenic protein (for example an albumin such as human serum albumin, e.g. as described in US-A-4718433, US-A-4774958, US-A-4844882, EP-A-0359246, WO-A-9112823, WO-A-9205806, WO-A-9217213, WO-A-9406477, WO-A-9501187 or WO-A-9638180) or protein as described in WO-A-9501187 or WO-A-9746264, a polymer material (for example a synthetic biodegradable polymer as described in EP-A-0398935, a synthetic polymer as described in US-A-5611344, a modified polymer as described in WO-A-9402106, an elastic interfacial synthetic polymer membrane as described in EP-A-0458745, a microparticulate biodegradable polyaldehyde as described in EP-A-0441468, a microparticulate N-dicarboxylic acid derivative of a polyamino acid - polycyclic imide as described in EP-A-0458079, or a biodegradable polymer as described in WO-A-9317718 or WO-A-9607434), a lipid, protein or polymer material as described in WO-A-9640285 or WO-A-9748337, a non-polymeric and non-polymerisable wall-forming material (for example as described in WO-A-9521631), or a surfactant (for example a polyoxyethylene-polyoxypropylene block copolymer surfactant such as a Pluronic, a polymer surfactant as described in WO-A-9506518, a film-forming surfactant such as a phospholipid, e.g. as described in WO-A-9211873, WO-A-9217212, WO-A-9222247, WO-A-9409829, WO-A-9428780, WO-A-9503835 or WO-A-9729783, or by a surfactant from the extensive list contained in EP-A-0727225 or as described in WO-A-9416739, a lipid as describe in WO-A-9428874 or WO-A-9428873, a lyophilised lipid as described in WO-A-9740858, a pressure resistant barrier forming material as described in WO-A-9640283, a fluorine containing amphiphilic material as in WO-A-9604018 or one or more lipopeptides).

Hollow inorganic microparticles, for example comprising calcium carbonate, quartz, alumina, nickel hydroxide or ferric hydroxide, may also be useful.

In one preferred embodiment of the invention, the encapsulating material comprises one or more phospholipids and/or lipopeptides. Thus, not only do gas microbubbles encapsulated by such materials tend to exhibit particularly high stability, but their molecules normally contain reactive moieties onto which targeting vectors such as antibodies or other affinity ligands may be attached, e.g. through an appropriate linking group.

Representative examples of useful phospholipids include lecithins (i.e. phosphatidylcholines), for example natural lecithins such as egg yolk lecithin or soya bean lecithin and synthetic or semisynthetic lecithins such as dimyristoylphosphatidylcholine, dipalmitoylphosphatidylcholine or distearoylphosphatidylcholine; phosphatidic acids; phosphatidylethanolamines; phosphatidylserines including saturated (e.g. hydrogenated or synthetic) natural phosphatidylserine and synthetic or semisynthetic dialkanoylphosphatidylserines such as distearoylphosphatidylserine, dipalmitoylphosphatidylserine and diarachidoylphospahtidylserine; phosphatidylglycerols; phosphatidylinositols; cardiolipins; sphingomyelins; fluorinated analogues of any of the foregoing; mixtures of any of the foregoing and mixtures with other lipids such as cholesterol.

A more detailed discussion of phospholipids useful for stabilising gas microbubbles is contained in WO-A-9729783.

Lipopeptides which may be used include lipid-substituted peptide moieties which are amphiphilic and capable of membrane formation. Such lipopeptides may be formed from individual peptide units each comprising from 2 to 50 amino acid residues and each carrying one or more lipophilic hydrocarbon chains containing between 5 and about 50 carbon atoms.

The number of amino acid residues in the individual peptide units is preferably less than 20, more preferably less than 10, and most preferably between 2 and 8. Clearly, keeping the number of amino acid residues to a minimum will both reduce costs and allow easier preparation of the lipopeptides.

In principle, any amino acid residues may be used in the preparation of individual peptide units, provided that the end product lipopeptide is amphiphilic. In a preferred embodiment, however, the peptide units comprise residues of the readily available twenty naturally occurring essential amino acids.

In one embodiment the peptide units may comprise alternating hydrophobic and hydrophilic amino acid residues such as alanyl and diaminopropionyl, and may comprise one or more complementary sequences and/or a targeting sequence with affinity for biological receptors. In a preferred embodiment, residues of charged amino acids such as lysine and glutamic acid are selected to provide side-chain functionalities comprising positively and/or negatively charged groups respectively at neutral pH. Although not wishing to be limited by theory, it is envisaged that these charged groups may help in stabilisation of the outer parts of membranes by forming ion-pairs or salt bridges. The alignment of oppositely charged groups leading to membrane stability is possible only if the peptide sequences involved are complementary to one another.

The lipid component of the lipopeptides preferably comprises an alkyl, alkenyl or alkynyl chain, especially an alkyl chain. Such chains preferably contain between 5 and 25 carbon atoms and most preferably are obtainable from readily available fatty acid derivatives. Suitable fatty acids include oleic acid, stearic acid, palmitic acid and the like; such fatty acids are well-known to the person skilled in the art. The number of hydrocarbon chains per individual lipopetide unit may vary depending on the number of residues present and may readily be determined by the person skilled in the art; typically each lipopeptide molecule will comprise one or two hydrocarbon chains.

The use of gas microbubbles in which the encapsulating membranes bear a net overall charge, for example owing to the presence of charged stabilising materials such as appropriate phospholipids or lipopeptides, may be advantageous in terms of enhancing the stability and dispersibility of the microbubbles, as well as their resistance to coalescence, thereby avoiding the need to use stabilising additives.

Membrane-forming amphiphilic encapsulating materials such as phospholipids and lipopeptides may be present at the microbubble-sample liquid interfaces as monolayers, bilayers or multilayers (e.g. comprising a plurality of bilayers).

In principle any substances, including mixtures, which are at least partially, e.g. substantially or completely, in gaseous or vapour form at typical processing temperatures (e.g. approximately 20°C) may be used as the microbubble gas. Representative gases thus include air; nitrogen; oxygen; carbon dioxide; hydrogen; inert gases such as helium, argon, xenon or krypton; sulphur fluorides such as sulphur hexafluoride, disulphur decafluoride or trifluoromethylsulphur pentafluoride; selenium hexafluoride; optionally halogenated silanes such as methylsilane or dimethylsilane; low molecular weight hydrocarbons (e.g. containing up to 7 carbon atoms), for example alkanes such as methane, ethane, a propane, a butane or a pentane, cycloalkanes such as cyclopropane, cyclobutane or cyclopentane, alkenes such as ethylene, propene, propadiene or a butene, and alkynes such as acetylene or propyne; ethers such as dimethyl ether; ketones; esters; halogenated low molecular weight hydrocarbons (e.g. containing up to 7 carbon atoms); and mixtures of any of the foregoing. Advantageously at least some of the halogen atoms in halogenated gases are fluorine atoms; thus biocompatible halogenated hydrocarbon gases may, for example, be selected from bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethylfluoride, 1,1-difluoroethane and perfluorocarbons. Representative perfluorocarbons include perfluoroalkanes such as perfluoromethane, perfluoroethane, perfluoropropanes, perfluorobutanes (e.g. perfluoro-n-butane, optionally in admixture with other isomers such as perfluoro-iso-butane), perfluoropentanes, perfluorohexanes or perfluoroheptanes; perfluoroalkenes such as perfluoropropene, perfluorobutenes (e.g. perfluorobut-2-ene), perfluorobutadiene, perfluoropentenes (e.g. perfluoropent-1-ene) or perfluoro-4-methylpent-2-ene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane or perfluorocycloheptane. Other halogenated gases include methyl chloride, fluorinated (e.g. perfluorinated) ketones such as perfluoroacetone and fluorinated (e.g. perfluorinated) ethers such as perfluorodiethyl ether. The use of perfluorinated gases, for example sulphur hexafluoride and perfluorocarbons such as perfluoropropane, perfluorobutanes, perfluoropentanes and perfluorohexanes, may be particularly advantageous in view of the recognised high stability of microbubbles containing such gases. Other gases with physicochemical characteristics which cause them to form highly stable microbubbles may likewise be useful. It will be appreciated that gases from the above list which boil above the intended separation processing temperature will in general be employed as components of mixtures with other more volatile gases rather than be used alone.

The size of microbubbles used in the process of the invention may vary depending on, for example, parameters such as the nature and size of the target to be isolated. Any microbubble which, when bound to the target, is less dense than the liquid medium of the sample may be suitable. For in vitro use the microbubbles may, for example, have diameters of from 50 nm to 50 µm, preferably between 200 nm and 25 µm. The microbubbles may conveniently be of similar size to the targeted components; thus, for example, if the target component is a cell, the microbubbles may have diameters of 1 to 10 µm, preferably 3 to 5 µm.

The number of microbubbles required to isolate a particular target component from a sample will vary depending on factors such as the natures of the microbubbles and the target, and particularly the number of other components present in the sample. In general, simple experimentation may be carried out to determine optimum microbubble:targeted component number ratios for particular separation systems in order to ensure flotation of the microbubble/targeted component complexes. At a more specific level the number of microbubbles required may also depend on the content of target component relative to non-target components in a sample; thus, for example, samples containing a low proportion of target component may require treatment with a relatively large number of microbubbles to ensure adequate separation.

The process of the invention may in principle be used to separate any target component which is suitable for separation from a liquid sample using floatable microbubbles. The target component may be similar in size to the microbubbles of the invention or may be larger or smaller; preferably, however, its size is less than 50 µm in diameter. Suitable target components may, for example, include metals (including metal ions and heavy metals), polymers, lipids, carbohydrates, blood components, proteins, glycoproteins, peptides (including prions), glycopeptides, hormones, immobilised combinatorial library components, cells and modified (e.g. transfected and/or infected) cells, fragments of cells, cell organelles, DNA, RNA, phages, enzymes, ribosomes, toxins, living organisms such as bacteria and viruses (including modified, e.g. transfected, bacteria and viruses) etc.

The process of the invention is particularly suitable for the separation of cells and other biological components derived from human or non-human animal subjects. Representative target cells include all CD positive cells such as antigen presenting cells (e.g. langerhans cells, endothelial cells, trophoblasts, neural cells and epithelial cells, including epithelial tumour cells which are markers of cancer not otherwise found in blood), hematopoietic cells (e.g. lymphocytes, granulocytes, monocytes, macrophages, reticulocytes and other cells expressing the transferrin receptor, erythrocytes, megakaryocytes and platelets), NK cells, hematopoietic progenitor cells, leukocytes, myeloid cells, modified (e.g. transfected) cells etc. In particular, the process of the invention may be employed to separate hematopoietic progenitors and/or stem cells from bone marrow/blood suspensions; such separations are important in the treatment of patients undergoing high dosage chemotherapy and are currently performed using far more complex and cumbersome techniques. Cell separation techniques according to the invention are also particularly effective in the removal or "purging" of cancer cells from a sample to yield a sample free of cancerous cells.

Binding of microbubbles to biological target components such as cells (including cancer cells, virus-infected cells and mycoplasma-infected cells), cell cultures, bacteria or viruses may also be used as a means of detecting the presence of such components in samples and may be used in methods of diagnosis. Thus, for example, microbubbles coupled to a vector which is specific for a particular disease marker component may be used to allow detection of that component in a sample. It may not be necessary to separate the floating microbubbles/target complexes from the sample since the presence of microbubble-bound target components such as cells, bacteria or viruses may be determined by techniques such as microscopy or flow cytometry, thereby allowing a physician or other skilled artisan to make an informed diagnosis based on the presence or absence of the target component.

In accordance with this embodiment of the invention there is therefore provided a diagnostic method for the detection of a disease marker component in a liquid sample, e.g. a blood or other body fluid sample, which comprises admixing said sample with encapsulated gas microbubbles capable of targeting said disease marker component, allowing said microbubbles and any coupled disease marker component to float to the surface of the sample to form a floating microbubble layer, and analysing said layer, e.g. by microscopy or flow cytometry, for the presence of said disease marker component.

In order to render them targetable, the microbubbles used in accordance with the invention may be coupled to one or more appropriate targeting moieties, e.g. chelating moieties, affinity ligands or vectors, either directly or through appropriate linking groups. In certain instances, however, the microbubble membranes may themselves have affinity for a target component and thus may be regarded as combined membranes and targeting moieties. One example of this is the use of phosphatidylserine-encapsulated gas microbubbles doped with a thiolated lipopeptide in the separation of hematopoietic progenitor cells. Thus it has been found that such microbubbles, when added to a population of murine bone marrow cells devoid of differentiated cells (i.e. so-called lineage negative cells), bind to and float a subpopulation which comprises less than 5% of the lineage negative cells but contains almost 100% of the *in vitro* colony forming cells, with more than every second cell in the separated population being a progenitor cell.

For non-biological targets such as metal ions, a chelating agent which binds particular metal ions may be attached to the microbubbles. Microbubbles may also be selected or modified so that they contain a reactive functional group designed to react with a particular complementary functional group in a target component. For example, microbubbles may carry an anhydride or acyl chloride moiety which will react readily with an amino or alcohol functionality on a target component. After flotation and separation the formed amide or ester bond may be broken using conventional techniques to release the target component.

In the separation of biological components, the microbubbles may, for example, be directly coupled to vectors such as monoclonal antibodies which recognise specific target components. Alternatively, the microbubbles may be coupled or linked to a peptide or a secondary antibody which has specificity for a primary antibody which in turn has specificity for the target components. Such use of secondary antibodies is advantageous in that appropriate selection of a secondary antibody allows the preparation of "universal" microbubbles which may be used for a wide range of applications since the primary antibody can be tailored to the particular target components.

The microbubbles may also be coupled or linked to substances such as streptavidin/avidin to allow biotinylated vectors to be coupled, or may be coupled or linked to vectors such as proteins, lectins, polysaccharides, peptides, nucleotides, carbohydrates, low molecular weight receptor agonists or antagonists, for example as known in the art. The use of vectors complementary to a functionality present in a target component will be readily achieved by the skilled artisan.

Functionalised microbubbles carrying one or more reactive groups may be employed in the process of the invention for binding to receptor molecules located on cell surfaces. Microbubbles comprising a thiol moiety, for example, may bind to cell surface receptors via disulphide exchange reactions. The reversible nature of such reactions means that coupling and subsequent detachment may be controlled by altering the redox environment. Similarly, functionalised microbubbles with membranes comprising activated esters such as N-hydroxysuccinimide esters may be used to react with amino groups found on a variety of cell surface molecules.

Coupling of a microbubble to a desired vector may be achieved by covalent or non-covalent means, for example involving interaction with one or more functional groups located on the microbubble and/or vector. Examples of chemically reactive functional groups which may be employed for this purpose include amino, hydroxyl, sulfhydryl, carboxyl, and carbonyl groups, as well as carbohydrate groups, vicinal diols, thioethers, 2-aminoalcohols, 2-aminothiols, guanidinyl groups, imidazolyl groups and phenolic groups. The vector and microbubble may also be linked via a linking group; many such groups are well known in the art. Connection of the vector and microbubble, optionally via a linker, may therefore be readily achieved using routine techniques, for example as summarised in WO-A-9818501, the contents of which are incorporated herein by reference.

The rate at which microbubbles and coupled target float to the surface of a sample may, if desired, be increased by subjecting the sample to centrifugation, for example using any appropriate, e.g. conventional centrifugation apparatus.

Separation of a floating microbubble/target component layer from a sample may for example by achieved by decantation, transfer from one syringe to another, or by simply skimming off the floating microbubble layer. If desired the separation may be enhanced by overlaying the sample with an immiscible low density fluid into which the microbubble/target component complexes will float, thereby totally separating them from the sample; two phase systems comprising immiscible aqueous layers with different densities, for example two phase dextran-polyethylene glycol-water systems, are desribed by Albertson in Partition of Cell Particles and Macromolecules (Wiley-Interscience, Second Edition,1971), e.g. at pp. 44-47.

It is an advantage of the invention that this component separation process does not require repeated time-consuming washing techniques in order to isolate target components such as cells.

Following separation, microbubble removal may, for example, be effected by bursting the microbubbles, for example by transient application of an overpressure or underpressure, by ultrasonication or by pH change. It will be appreciated that the microbubble bursting conditions should be sufficiently mild to avoid damaging the components; tests have shown that brief overpressures of up to 2-4 atmospheres may be used to destroy microbubbles without harming target components such as cells in separated products. Following microbubble removal by bursting, a proportion of microbubble-encapsulating material may remain attached to the separated target component; with regard to the separation of cells this will in general be insufficient to affect the viability of the cells. The remainder of the microbubble encapsulating material may if desired be removed by, for example, simple washing procedures.

One useful method for applying a specific overpressure or underpressure of A atmospheres to a separated microbubble/target component fraction is to introduce the fraction into a syringe and then draw in a volume V of air or another (e.g. inert) gas. The tip of the syringe is then closed, for example by means of an appropriate tap, whereafter the plunger is briefly moved so as to change the gas volume to V/A, at which point an overpressure or underpressure of A atmospheres will be present in the syringe provided that a constant temperature is maintained.

Other microbubble removal techniques which may be employed, e.g. when using gas microbubbles which are relatively resistant to bursting, include uncoupling/detaching the microbubbles by, for example, hydrolysis, pH change, salt addition, change in redox environment, enzymatic cleavage etc.

It has been found that the viability of certain target components, e.g. cells, obtained in accordance with the invention is often unaffected by the presence of the attached biocompatible microbubbles, so that such components may be manipulated further, e.g. cultured, prior to the microbubble removal step.

Owing to the ease with which microbubble removal may be achieved and the fact that biocompatible gas microbubbles may be employed, the present process is particularly suitable for positive component selection in the biological and biotechnological fields.

Thus, for example, cells transfected or cotransfected with membrane-associated proteins may be separated from untransfected cells using microbubbles coupled to a vector with affinity for the membrane-associated protein. This may advantageously be used during transient transfection of cells to establish cell cultures with a high density of transfected cells, which is often essential for determining the effect of a transfected gene.

Microbubbles coupled to an affinity ligand such as a protein, peptide, oligonucleotide, carbohydrate, ion exchange material, metal binding moiety or low molecular weight receptor agonist or antagonist may be used to concentrate and purify molecules of interest from a crude biological sample such as a fermentation broth. Such concentration and purification may thereby be achieved in a single step without the use of expensive and cumbersome equipment such as centrifuges, filters and large-scale chromatography columns. After removal of the microbubbles/affinity ligand, the molecules of interest may be separated from any remaining microbubbles by removing the latter in a further flotation separation, by extraction (the microbubble components often being substantially more hydrophobic than the molecules of interest) or by any appropriate conventional purification method.

Microbubbles coupled to a protein may be used in the screening ("biopanning") of a phage display library. The protein may, for example, be obtained by culturing cells transfected with cDNA encoding the particular protein. cDNA encoding a protein of interest may be ligated in a plasmid in frame with a secretory signal and a tag (e.g. six histidines or an antibody-binding peptide, maltose-binding peptide or calmodulin-binding peptide) and the plasmid used to transfect a suitable cell line. Microbubbles coupled to an appropriate vector (e.g. Ni²⁺, antibody, maltose or calmodulin) will bind the secreted recombinant protein, and may then be used directly in biopanning processes to isolate peptides with affinity for the protein. A similar approach may be used to identify ligands from other libraries, such as combinatorial libraries or any other peptide display library. This method may be particularly useful as a tool to find antibody antagonists, e.g. of use in diminishing unwanted immunological reactions such as autoimmune disorders.

Immobilised combinatorial peptide libraries bound to solid-phase supports such as polymer beads may also be investigated. Such solid-phase polymer-supported libraries typically comprise millions of porous spherical beads of a size comparable to that of gas microbubbles used in accordance with the present invention. Following incubation of targeted microbubbles with such an immobilised combinatorial library, support beads carrying binding peptides will float to the surface with the microbubbles, while beads with non-binding sequences will sink to the bottom of the incubation vessel under the effect of gravity. Microbubble-bound polymer beads may be separated by decantation, whereafter the microbubbles may be removed and residual microbubble material may be washed away. The thus-obtained peptide may then be subjected to analysis by, for example, micro-sequencing or tandem mass spectrometry in order to identify the binding sequence.

According to a further embodiment of the invention there is provided apparatus for use in the separation of components of a liquid sample by flotation, said apparatus comprising two chambers interconnected such that a microbubble-containing sample may be drawn into one chamber and microbubble/target component complexes may be allowed to float and then transferred into the other chamber.

Preferably, such apparatus comprises two opposed variable volume chambers such as syringes connectable to each other and to microbubble and sample source via a 3-way valve. Microbubbles and sample are initially introduced into the first syringe, where the microbubbles bind to their target. The syringe is positioned vertically with its plunger downwards, so that the microbubble/target complexes float to the needle end whilst unbound components remain at the bottom of the syringe; the rate at which flotation proceeds may, if desired, be increased by use of appropriate centrifugation apparatus to apply centrifugal force. The floating microbubble/target complexes are transferred to the second syringe by withdrawal of the plunger of the second syringe, thereby effecting ready separation of bound and unbound components. In a negative selection, the desired component(s) will be in the first syringe and can be isolated without further manipulation. In a positive selection, the desired component(s) will be in the second syringe attached to the microbubbles; a short, gentle pressure may be applied to the plunger of the second syringe in order to burst the microbubbles, whereafter the desired components may be removed from the second syringe.

According to a still further embodiment of the invention there is provided apparatus for use in continuous flow separation of components of a sample by flotation in accordance with the method of the invention, said apparatus comprising feed means adapted to supply a continuous flow of targeted encapsulated gas microbubble-containing sample to a separation vessel having a downwardly converging side or sides (e.g. as in an inverted cone or pyramid), said vessel being equipped at its bottom with means for withdrawing sample liquid and unbound sample components and at its top with means permitting the overflow of sample liquid and microbubble/target component complexes. Either or both of the separated fractions may be collected for further processing, as appropriate.

In the accompanying drawings, which serve to illustrate the invention without in any way limiting the same:
Fig. 1 is a schematic representation of the use of two interconnected syringes in a flotation separation; and
Fig. 2 is a side view of apparatus useful in a continuous flow separation process according to the invention.

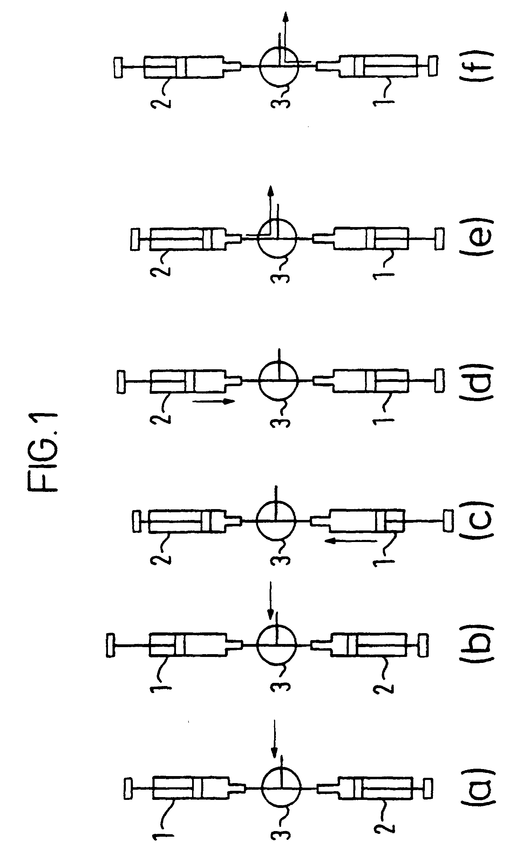

Referring to Fig. 1 in more detail, the apparatus comprises diametrically opposed syringes 1 and 2, which are connectable to each other and to sources of microbubbles and sample (not shown) through three-way valve 3. At (a) microbubbles are drawn into syringe 1, and at (b) sample is drawn in. As shown at (c) the apparatus is then rotated through 180° to enhance mixing of the microbubbles and sample, and microbubble/target complexes are allowed to float to the top of syringe 1 and then transferred to syringe 2. As shown at (d) pressure may then be applied to the plunger of syringe 2, following closure of valve 3, in order to burst the microbubbles; thereafter the separated sample component may be expelled from syringe 2 as shown at (e). Alternatively, in a negative selection procedure, the desired sample component is expelled from syringe 1, as shown at (f).

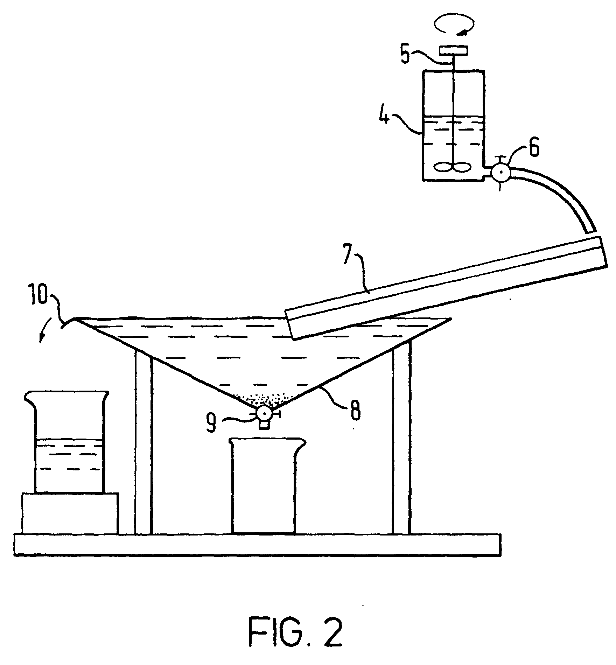

Referring to Fig. 2, microbubbles and sample are mixed in feedbox 4 equipped with stirrer 5, and are fed through valve 6 to channel 7 and thence into the conical separation vessel 8. Vessel 8 is fitted with a tailing removal valve 9 at its base and has a lip 10 permitting overflow of sample liquid and floating microbubble/target complexes.

The following non-limiting examples serve to illustrate the invention.

### Example 1

### Preparation of gas microbubbles encapsulated with DSPS and thiolated anti-CD34-Mal-PEG₂₀₀₀-DSPE, useful for separation of haematopoietic stem cells

### a) Synthesis of Boc-NH-PEG₂₀₀₀-DSPE [t-butyl carbamate poly(ethylene glycol)distearoylphosphatidylethanolaminel

Distearoylphosphatidylethanolamine (DSPE - 31 mg) was added to a solution of Boc-NH-PEG₂₀₀₀-SC (150 mg) in chloroform (2 ml), followed by triethylamine (33 µl). The mixture was stirred at 41°C for 10 minutes until the starting material had dissolved. The solvent was rotary evaporated and the residue was taken up in acetonitrile (5 ml). The resulting dispersion was cooled to 4°C and centrifuged, whereafter the solution was filtered and evaporated to dryness. The structure of the resulting product was confirmed by NMR.

### b) Synthesis of H₂N-PEG₂₀₀₀-DSPE [amino-poly(ethylene glycol)distearoylphosphatidylethanolamine]

Boc-NH-PEG₂₀₀₀-DSPE (167 mg) was stirred in 4 M hydrochloric acid in dioxane (5 ml) for 2.5 hours at ambient temperature. The solvent was removed by rotary evaporation and the residue was taken up in chloroform (1.5 ml) and washed with water (2 x 1.5 ml). The organic phase was evaporated *in vacuo*. TLC analysis (chloroform/methanol/water 13:5:0.8) gave a single ninhydrin positive spot with Rf = 0.6; confirmation of the structure was obtained by NMR.

### c) Synthesis of Mal-PEG₂₀₀₀-DSPE [3-maleimidopropionate poly(ethylene glycol)distearoylphosphatidylethanolamine]

A solution of N-succinimidyl-3-maleimidopropionate (5.6 mg, 0.018 mmol) in tetrahydrofuran (0.2 ml) was added to H₂N-PEG₂₀₀₀-DSPE (65 mg, 0.012 mmol) dissolved in tetrahydrofuran (1 ml) and 0.1 M sodium phosphate buffer pH 7.5 (2 ml). The mixture was warmed to 30°C and the reaction was followed to completion by TLC, whereafter the solvent was removed *in vacuo*. The title material was purified on a flash silica column using 80:20 chloroform:methanol as eluent. The structure of the pure product was confirmed by NMR and mass spectrometry.

### d) Preparation of gas microbubbles encapsulated with DSPS "doped" with Mal-PEG₂₀₀₀-DSPE

Distearoylphosphatidylserine (DSPS - 4.5 mg) and Mal-PEG₂₀₀₀-DSPE from (c) above (0.5 mg) were weighed into a clean vial and 1 ml of a solution of 1.4% propylene glycol/2.4% glycerol was added. The mixture was warmed to 80°C for 5 minutes and then filtered through a 4.5 µm filter. The sample was cooled to room temperature and the head space was flushed with perfluorobutane gas. The vial was shaken in a cap mixer for 45 seconds and the resulting microbubbles were washed three times with distilled water.

### e) Thiolation of anti-CD34 antibodies

To 0.3 mg of anti-CD34 antibody dissolved in 0.5 ml phosphate buffered saline (PBS), pH7, was added 0.3 mg Traut's reagent and the solution was stirred at room temperature for 1 hour. Excess reagent was separated from the modified protein on a NAP-5 column.

### f) Conjugation of thiolated anti-CD34 antibody to gas microbubbles encapsulated with DSPS and Mal-PEG₂₀₀₀-DSPE

0.5 ml of the thiolated antibody praparation from (e) was added to an aliquot of microbubbles from (d) and the conjugation reaction was allowed to proceed for 30 minutes on a roller table. Following centrifugation at 2000 rpm for 5 minutes the infranatant was removed. The microbubbles were washed a further three times with water.

### g) Detection of the antibody conjugated with the microbubbles using a FITC-conjugated secondary antibody

To the microbubble suspension from (f) was added 0.025 ml FITC-conjugated goat anti-mouse antibody. The mixture was incubated in the dark at room temperature for 30 minutes on a roller table and was then centrifuged at 2000 rpm for 5 minutes. The infranatant was then removed and the microbubbles were washed a further three times with water. Flow cytometric analysis of the microbubble suspension showed that 98% of the population was fluorescent.

### h) Separation of CD34 positive cells

White blood cells are collected by centrifugal elutrition from a patient injected daily for four days with G-CSF (10 µg/day). Then microbubbles with CD34 antibodies are mixed with the cells in a ratio of 10:1 in a centrifuge tube and placed on a roller mixer for 30 minutes. The tube is then centrifuged for 5 minutes at 400 x g and the cells bound to microbubbles floating on the top are collected. The collected suspension is subjected to a pressure sufficient to break the bubbles without harming the cells, and the cells are then transplanted to a patient in need for such cells.

### Example 2

### Preparation of gas microbubbles encapsulared with DSPS and thiolated anti-CD62-Mal-PEG₂₀₀₀-DSPE

An identical procedure to that described in Example 1 was used to prepare microbubbles comprising anti-CD62 antibodies.

The microbubbles are used to separate CD62 positive cells from CD62 negative cells or from cells with a low expression of the antigen.

### Example 3

### Preparation of gas microbubbles encapsulated with DSPS and thiolated anti-ICAM-1-Mal-PEG₂₀₀₀-DSPE

An identical procedure to that described in Example 1 was used to prepare microbubbles comprising anti-ICAM-1 antibodies.

The microbubbles are used to separate ICAM-1 positive cells from ICAM-1 negative cells or from cells with a low expression of the antigen.

### Example 4

### Preparation of gas microbubbles encapsulated with DSPS, thiolated anti-CD62-Mal-PEG₂₀₀₀-DSPE and thiolated-anti-ICAM-1-Mal-PEG₂₀₀₀-DSPE

This example describes the preparation of microbubbles comprising multiple antibody vectors for separating cells expressing both antigens from negative cells or cells expressing one antigen only.

### a) Preparation of gas microbubbles encapsulated with DSPS and Mal-PEG₂₀₀₀-DSPE

DSPS (4.5 mg) and Mal-PEG₂₀₀₀-DSPE from Example 1 (0.5 mg) were weighed into a clean vial and 1 ml of a solution of 1.4% propylene glycol/2.4% glycerol was added. The mixture was warmed to 80°C for 5 minutes and then filtered through a 4.5 µm filter. The sample was cooled to room temperature and the head space was flushed with perfluorobutane gas. The vial was shaken in a cap mixer for 45 seconds and the resulting microbubbles were washed three times with distilled water.

### b) Thiolation of anti-CD62 and anti-ICAM-1 antibodies

To 0.3 mg each of anti-CD62 and anti-ICAM-1 antibodies dissolved in PBS buffer (pH 7, 0.5 ml) was added Traut's reagent and the solutions were stirred at room temperature for 1 hour. Excess reagent was separated from the modified protein on a NAP-5 column.

### c) Conjugation of thiolated anti-CD62 and anti-ICAM-1 antibodies to gas microbubbles encapsulated with DSPS and Mal-PEG₂₀₀₀-DSPE

0.5 ml of the mixed thiolated antibody preparation from (b) was added to an aliquot of microbubbles from (a) and the conjugation reaction was allowed to proceed for 30 minutes on a roller table. Following centrifugation at 2000 rpm for 5 minutes, the infranatant was removed. The microbubbles were washed a further three times with water.

The PEG spacer length may be varied to include longer (e.g. PEG₃₄₀₀ and PEG₅₀₀₀) or shorter (e.g. PEG₆₀₀ or PEG₈₀₀) chains. Addition of a third antibody such as thiolated-anti-CD34 is also possible.

### Example 5

### Preparation of transferrin-coated gas microbubbles for separation of cells with high expression of transferrin receptors

### a) Synthesis of a thiol-functionalised lipid molecule

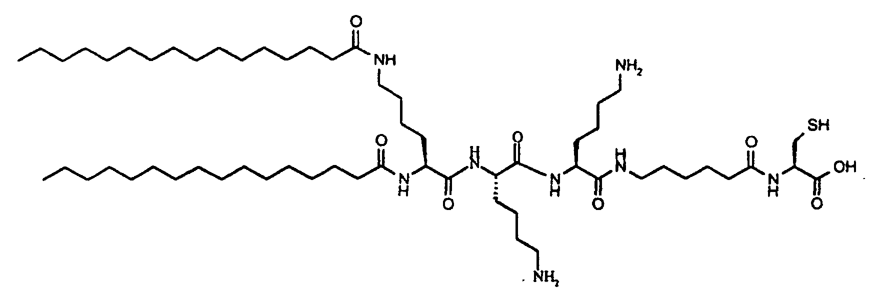

The lipid structure shown above was synthesised on an ABI 433A automatic peptide synthesiser starting with Fmoc-Cys(Trt)-Wang resin on a 0.25 mmol scale, using 1 mmol amino acid cartridges. All amino acids and palmitic acid were preactivated using *O*-benzotriazol-1-yl-*N*,*N*,*N*',*N*',-tetramethyluronium hexafluorophosphate (HBTU) before coupling. Simultaneous removal of peptide from the resin and deprotection of side-chain protecting groups was carried out in trifluoroacetic acid (TFA) containing 5% 1,2-ethanedithiol (EDT) and 5% water for 2 hours, giving a crude product yield of 250 mg. Purification by preparative HPLC of a 40 mg aliquot of crude material was carried out using a gradient of 90 to 100% B over 50 minutes (A = 0.1% TFA/water and B = methanol) at a flow rate of 9 ml/minute. After lyophilisation, 24 mg of pure material was obtained (analytical HPLC: gradient 70-100% B where B = 0.1% TFA/acetonitrile, A = 0.01% TFA/water; detection - UV 214 nm; product retention time = 23 minutes). Further product characterisation was carried out using MALDI mass spectrometry: expected M+H at 1096, found at 1099.

### b) Preparation of gas microbubbles encapsulated with DSPS "doped" with a thiol-containing lipid structure

DSPS (4.5 mg) and lipid from (a) above (0.5 mg, 0.4 mmol) were weighed into a clean vial and 0.8 ml of a solution of 1.4% propylene glycol/2.4% glycerol was added. The mixture was warmed to 80°C for 5 minutes (vial shaken during warming) and filtered while still hot through a 40 µm filter. The sample was cooled to room temperature and the head space was flushed with perfluorobutane gas. The vial was shaken in a cap mixer for 45 seconds and then placed on roller table overnight. The resulting microbubbles were washed several times with deionised water and analysed for thiol group incorporation using Ellmans Reagent.

### c) Modification of transferrin with fluorescein-NHS and Sulpho-SMPB

To 4 mg of transferrin (Holo, human) in PBS (1 ml) was added 0.5 ml dimethylsulphoxide solution containing 1 mg Sulpho-SMPB and 0.5 mg fluorescein-NHS. The mixture was stirred for 45 minutes at room temperature and then passed through a Sephadex 200 column using PBS as eluent. The protein fraction was collected and stored at 4°C prior to use.

### d) Microbubble conjugation with transferrin

To the thiol-containing microbubbles from (b) was added 1 ml of the modified transferrin protein solution from (c). After adjusting the pH of the solution to 9 the conjugation reaction was allowed to proceed for 2 hours at room temperature. Following extensive washing with deionised water the microbubbles were analysed by Coulter counter (97% between 1 and 5 µm) and fluorescence microscopy (highly fluorescent microbubbles were observed).

### e) Separation of transferrin receptor positive cells

Microbubbles with transferrin form (d) are added to proliferating cells (U 937, ATCC] in a centrifuge tube in a ratio of 10:1 and the tube is placed at 37°C on a roller mixer for 30 minutes. The tube is then centrifuged for 5 minutes at 200 x g and the cells bound to microbubbles floating on the top are collected. The cells are analysed by flow cytometry after disrupting the microbubbles with a gentle overpressure to determine the percentage of transferrin receptor positive cells.

### Example 6

### Preparation of gas microbubbles carrying antibodies to heat stable enterotoxin (ST-peptide), for purifying the peptide after fermentation

Antibodies to the ST-peptide are obtained after immunising sheep with the peptide conjugated to a suitable carrier. The antibodies are coupled to gas microbubbles using the same procedure as outlined for transferrin in Example 5(d). These microbubbles are added to the culture medium (1 µl of microbubble suspension per ml of culture medium) in which E. coli with the gene for the ST-peptide inserted have been cultured to produce and release optimal amounts of the peptide. The microbubbles floated after incubation for 30 minutes at room temperature followed by centrifugation at 200 x g for 5 minutes are collected, and the amount and function (receptor binding) of the peptide are detemined after detachment from the microbubbles at low pH.

### Example 7

### Preparation of gas microbubbles for purifying bioactive molecules

Antibodies to bioactive molecules, e.g. haematopoietic regulators such as G-CSF, GM-CSF and SCF are coupled to microbubbles as decribed for transferrin in Example 5(d). These microbubbles are added to a culture medium (1 µl of microbubble suspension per ml of culture medium) in which E. coli with the appropriate gene inserted has been cultured to produce and release optimal amounts of the proteins. The microbubbles floated after incubation for 30 minutes at room temperature followed by centrifugation at 200 x g for 5 minutes are collected and the amount and function (receptor binding) of the proteins are determined after detachment from the microbubbles at low pH.

### Example 8

### Preparation of gas microbubbles carrying goat anti-mouse antibody, for separation of cells tagged to epitopes on the cell surface with a primary mouse antibody

The microbubbles were prepared as decribed in Example 5(d) except that transferrin was replaced by goat anti-mouse antibody. Mononuclear cells were obtained after centrifugation of 10 ml of anticoagulated human peripheral blood through a density gradient and collection of the interphase cell layer. The cells were mixed with FITC-conjugated mouse anti-human CD4 antibodies, and the fraction of cells with bound antibodies after washing was determined by flow cytometry. The microbubbles carrying goat anti-mouse antibodies were then added to the cells in a centrifuge tube in a ratio of 10:1, and the tube was maintained at 37°C and placed on a roller mixer for 30 minutes. The tube was then centrifuged for 5 minutes at 400 x g and the cells bound to microbubbles floating at the top were collected. Analysis of the cells by flow cytometry after disrupting the microbubbles with a gentle overpressure in a syringe showed that 87% of the cells were CD4 positive. No cells were floated by microbubbles not carrying antibodies.

### Example 9

### Separation of hematopoietic stem cells mobilised to human peripheral blood with microbubbles carrying secondary antibodies

White blood cells collected by centrifugal elutrition from a patient injected daily for four days with granulocyte colony-stimulating factor (G-CSF) (10-15 µg/day) are mixed with antibodies which recognise the CD34 antigen on hematopoietic progenitor cell and incubated on ice for 30 minutes. Then microbubbles carrying antibodies directed against the CD34 antigen are mixed with the cells in a ratio of 10:1 in a centriguge tube and placed on a roller mixer for 30 minutes. The tube is then centrifuged for 5 minutes at 200 x g and the cells bound to microbubbles floating at the top are collected. The collected suspension is subjected to a pressure sufficient to break the microbubbles without harming the cells, and the cells are then transplanted to a patient in need for such cells.

### Example 10

### Isolation of haematopoietic stem cells from murine bone marrow

Bone marrow cells (BMC) from femur/tibia of C57bl/6J mice were obtained by flushing with MEM alpha culture medium. A cocktail of cell lineage specific antibodies directed against: CD2, CD8a, CD4, Mac-1, B220, Gr-1 and TER-119 (rat IgG isotype, PharMingen, San Diego, Ca) was added and the cells were incubated on ice for 30 minutes. Cells tagged with antibodies were removed with Dynabeads coated with sheep anti-rat IgG antibodies. The remaining lineage negative fraction was incubated with FITC-conjugated antibodies to Sca 1 (Ly 6A/E, clone E13-161.7, PharMingen) and sorted either by flow cytometric cell sorting or by incubating for 30 minutes with microbubbles prepared as in Example 8 but coated with goat anti-rat antibodies binding to the Sca 1 antibodies and collecting after flotation. The thus-obtained cell fractions were assayed for their content of high proliferative potential colony-forming cells (HPP-CFC) by culturing in agar dishes, 400 cells per dish, in the presence of appropriate growth factors (SCF, IL-1, IL-3, IL-6, IL-11, G-CSF and GM-CSF). The cell fraction isolated by flotation with microbubbles had a higher total number and concentration of stem cells forming large colonies (> 0.5 mm in diameter) than the cell population sorted in the flow cytometer. Also, smaller colonies were detected from cells isolated by the microbubble procedure whereas none developed from cells sorted in the flow cytometer.

### Example 11

### Preparation of gas microbubbles encapsulated with DSPS "doped" with a multiple-specific lipopeptide comprising a heparin sulphate-binding peptide (KRKR) and a fibronectin peptide (WOPPRARI)

This example describes the preparation of targeted microbubbles comprising multiple peptidic vectors arranged in a linear sequence for cell separation.

### a) Synthesis of a lipopeptide comprising a heparin sulphate-binding peptide (KRKR) and fibronectin peptide (WOPPRARI)

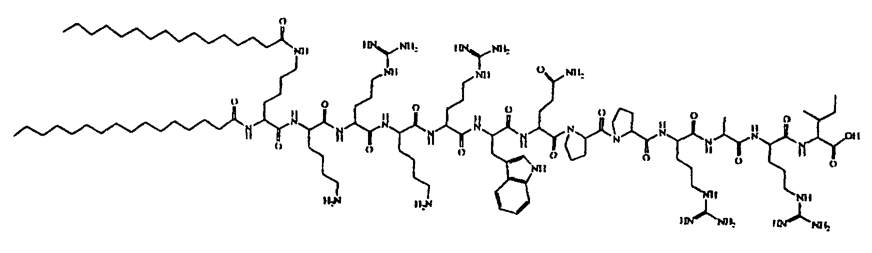

The above lipopeptide was synthesised on an ABI 433A automatic peptide synthesiser starting with Fmoc-Ile-Wang resin on a 0.1 mmol scale, using 1 mmol amino acid cartridges. All amino acids and palmitic acid were preactivated using HBTU before coupling. Simultaneous removal of peptide from the resin and side-chain protecting groups was carried out in TFA containing 5% phenol, 5% EDT, 5% anisole and 5% water for 2 hours, giving a crude product yield of 150 mg. Purification by preparative HPLC of a 40 mg aliquot of crude material was carried out using a gradient of 70 to 100% B over 40 minutes (A = 0.1% TFA/water and B = methanol) at a flow rate of 9 ml/minute. After lyophilisation, 16 mg of pure material were obtained (analytical HPLC: gradient 70-100% B where B = methanol, A = 0.01% TFA/water; detection - UV 260 and fluorescence, Ex₂₈₀, Em₃₅₀; product retention time = 19.44 minutes). Further product characterisation was carried out using MALDI mass spectrometry: expected M+H at 2198, found at 2199.

### b) Preparation of gas microbubbles encapsulated with DSPS "doped" with multiple-specific lipopeptide comprising a heparin sulphate-binding peptide (KRKR) and fibronectin peptide (WOPPRARI)

Samples of DSPS (4.5 mg) and lipopeptide from (a) (0.5 mg) were both weighed into each of two vials, and 0.8 ml of a solution of 1.4% propylene glycol/2.4% glycerol was added to each vial. The mixtures were warmed to 80°C for 5 minutes (vials shaken during warming). The samples were cooled to room temperature and the head spaces flushed with perfluorobutane gas. The vials were shaken in a cap mixer for 45 seconds and rolled overnight. The resulting microbubbles were washed several times with deionised water and analysed by Coulter counter [size: 1-3 µm (87%), 3-5 µm (11.5%)] and acoustic attenuation (frequency at maximum attenuation: 3.5 MHz). The microbubbles were stable at 120 mm Hg. MALDI mass spectral analysis was used to confirm incorporation of lipopeptide into the DSPS-encapsulated microbubbles as follows: ca. 0.05-0.1 ml of microbubble suspension was transferred to a clean vial and 0.05-0.1 ml methanol was added. The suspension was sonicated for 30 seconds and the solution was analysed by MALDI MS. Positive mode gave M+H at 2200 (expected for lipopeptide, 2198).

### Example 12

### Preparation of gas microbubbles encapsulated with DSPS "doped" with a lipopeptide comprising a helical peptide with affinity for cell membranes

This example describes the preparation of targeted microbubbles comprising a peptidic vector for targeting of cell membrane structures.

### a) Synthesis of lipopeptide comprising a helical peptide with affinity for cell membranes

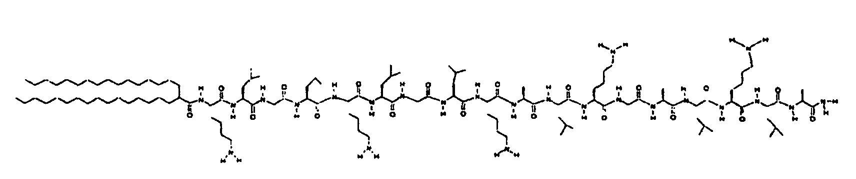

The above lipopeptide was synthesised on an ABI 433A automatic peptide synthesiser starting with Rink amide resin on a 0.2 mmol scale, using 1 mmol amino acid cartridges. All amino acids and 2-n-hexadecylstearic acid were preactivated using HBTU before coupling. Simultaneous removal of lipopeptide from the resin and side-chain protecting groups was carried out in TFA containing 5% water for 2 hours, giving a crude product yield of 520 mg. Purification by preparative HPLC of a 30 mg aliqout of crude material was carried out using a gradient of 90 to 100% B over 40 minutes (A = 0.1% TFA/water and B = methanol) at a flow rate of 9 ml/minute. After lyophilisation, 10 mg of pure material was obtained (analytical HPLC: gradient 90-100% B over 20 minutes where B = methanol, A= 0.01% TFA/water; detection - UV 214 nm; product retention time = 23 minutes). Further product characterisation was carried out using MALDI mass spectrometry: expected M+H at 2369, found at 2375.

### b) Preparation of encapsulated gas microbubbles

DSPS (4.5 mg) and lipopeptide from (a)(0.5 mg) were weighed into a clean vial and 1.0 ml of a solution of 1.4% propylene glycol/2.4% glycerol was added. The mixture was sonicated for 3-5 minutes, warmed to 80°C for 5 minutes and then filtered through a 4.5 µm filter. The mixture was cooled to room temperature and the head space was flushed with perfluorobutane gas. The vial was shaken in a cap mixer for 45 seconds and the resulting microbubbles were centrifuged at 1000 rpm for 3 minutes. The microbubbles were then washed with water until no lipopeptide could be detected in the wash water (MALDI-MS). Coulter counter, acoustic attenuation and pressure stability studies were performed. To confirm the presence of lipopeptide, methanol (0.5 ml) was adedd to an aliquot of the washed bubbles (ca. 0.2 ml) and the mixture was placed in a sonicator bath for 2 minutes. The resulting clear solution, on analysis by MALDI-MS, was found to contain the lipopeptide.

### Example 13

### Flotation of cell line ECV 304 by gas microbubbles carrying vectors which specifically bind thereto

The cell line ECV 304, derived from a normal umbilical cord (ATCC CRL-1998), originally thought to be a human endothelial cell line but now known to be a bladder carcinomal cell line, was cultured in Nunc culture flasks (Chutney 153732) in RPMI 1640 medium to which L-glutamine 200 mM, penicillin/streptomycin (10.000 U/ml and 10.00 mcg/ml) and 10% fetal calf serum had been added. The cells were subcultured following trypsination with a split ratio of 1:5 to 1:7 when reaching confluence. Two million cells from trypsinated confluent cultures were added to each set of five centrifuge tubes. Then control microbubbles or microbubbles capable of binding to endothelial cells (made as described in Examples 11 and 12) were added at 2, 4, 6, 8 or 10 million bubbles per tube. The cells at the bottom of the tubes after centrifugation at 400 x g for 5 minutes were counted with a Coulter counter. It was found that four or more microbubbles binding to a cell caused the cell to float to the top of the fluid in the centrifugation tube. All cells were floated by the microbubbles from Example 12 whereas about 50% were floated with the microbubbles from Example 11. The floated cells were separated from the sample by decantation or simply by skimming the floating microbubbles from the surface of the sample. Alternatively, the apparatus of Fig. 1 may be employed in the flotation and separation of endothelial cells by microbubbles.

### Example 14

### Gas microbubbles encapsulated with polymer from ethylidene bis(16-hydroxyhexadecanoate) and having adipoyl chloride and biotin-amidocaproate-Ala covalently attached to the polymer

### a) Synthesis of Z-Ala-polymer [3-O-(carbobenzyloxy-L-alanyl)polymer]

The polymer is prepared from ethylidene bis(16-hydroxyhexadecanoate) and adipoyl chloride as described in WO-A-9607434, and a polymer fraction with molecular weight 10,000 is purified using gel permeation chromatography. 10 g of the material (corresponding to 1 mmol OH groups), Z-alanine (5 mmol) and dimethylaminopyridine (4 mmol) are dissolved in dry dimethylformamide/tetrahydrofuran and dicyclohexylcarbodiimide is then added. The reaction mixture is stirred at ambient temperature overnight. Dicyclohexylurea is filtered off and the solvent is removed using rotary evaporation. The product is purified by chromatography, fractions containing the product are combined and the solvent is removed using rotary evaporation. The structure of the product is confirmed by NMR.

### b) Synthesis of Ala-polymer [3-O-(L-alanyl)-polymer]

Z-Ala-polymer (0.1 mmol) from (a) is stirred in toluene/tetrahydrofuran and glacial acetic acid (15% of the total volume) and hydrogenated in the presence of 5% palladium on charcoal for 2 hours. The reaction mixture is filtered and concentrated *in vacuo*.

### c) Synthesis of biotinamidocaproate-Ala-polymer

A solution of biotinamidocaproate N-hydroxysuccinimide ester in tetrahydrofuran is added to Ala-polymer from (b), dissolved in a mixture of tetrahydrofuran and dimethylformamide and 0.1 M sodium phosphate buffer having a pH of 7.5. The reaction mixture is heated to 30°C and stirred vigorously; the reaction is monitored to completion by TLC. The solvent is evaporated and the crude product is used without further purification.

### d) Gas microbubbles comprising biotin-amidocaproate-Ala-polymer and PEG 10000 methyl ether 16-hexadecanoyloxyhexadecanoate

10 ml of a 5% w/w solution of biotin-amidocaproate-Ala-polymer from (c),in (-)-camphene maintained at 60°C, is added to 30 ml of a 1% w/w aqueous solution of PEG 10000 methyl ether 16-hexadecanoyloxyhexadecanoate (prepared as described in WO-A-9607434) at the same temperature. The mixture is emulsified using a rotor stator mixer (Ultra Turax® T25) at a slow speed for several minutes, and thereafter is frozen in a dry ice/methanol bath and lyophilized for 48 hours, giving the title product as a white microparticulate powder.

### e) Microscopy characterisation of the product

Confirmation of the microparticulate nature of the product is performed using light microscopy as described in WO-A-9607434. Ultrasonic transmission measurements using a 3.5 MHz broadband transducer indicate that a microparticle suspension of less than 2 mg/ml gives a sound beam attenuation of at least 5 dB/cm.

### Example 15

### Gas microbubbles encapsulated with albumin and functionalised with biotin

A homogeneous suspension of gas-filled albumin microspheres (6x10⁸ microspheres/ml) in 5 mg/ml albumin was used, with all manipulations being carried out at room temperature. Two 10 ml aliquots were centrifuged (170 x g, 5 minutes) to promote flotation of the microspheres and 8 ml of the underlying infranatant was removed by careful suction and replaced by an equal volume of air-saturated phosphate buffered saline, the preparations being rotated for 15-20 minutes to resuspend the microspheres. This procedure was repeated twice, whereafter only negligible amounts of free non-microsphere-associated albumin were assumed to remain. 50 µl of NHS-biotin (10 mM in dimethylsulphoxide) was added to one of the aliquots (final concentration 50 µM); the other (control) aliquot received 50 µl of dimethylsulphoxide. The tubes containing the samples were rotated for 1 hour whereafter 20 µl portions of 50% aqueous glutaraldehyde were added to each tube to crosslink the microspheres. After rotation for another hour the tubes were positioned vertically overnight to allow flotation of the microspheres. The next day, the suspensions were washed twice with phosphate buffered saline containing 1 mg/ml human serum albumin (PBS/HSA) and were resuspended in PBS/HSA after the last centrifugation.

In order to determine the presence of microsphere-associated biotin, streptavidin conjugated to horseradish peroxidase (strep-HRP) was added to both suspensions and the tubes were rotated for 1 hour to allow for reaction. The microspheres were then washed three times, resuspended in 100 mM citrate-phosphate buffer (pH 5) containing 0.1 mg/ml phenylenediamine dihydrochloride and 0.01% hydrogen peroxide, and rotated for 10 minutes. Development of a yellow-green colour was indicative of the presence of enzyme. The following results were obtained:

| Sample | Colour development |
|---|---|
| Biotinylated microspheres + strp-HRP | 2+ |
| | |
| Control microspheres + strp-HRP | + |

This confirms that the microspheres were biotinylated.

### Example 16

### Separation of transfected U937-1 cells from non-transfected cells

The pHook-1 plasmid (Invitrogen, Groningen, Netherlands) encodes a single chain antibody (sFv) directed against the hapten phOx (4-ethoxymethylene-2-phenyl-2-oxazolin-5-one). The sFv is fused to a transmembrane region from the PDGF-receptor and will be expressed at the cell surface of transfected cells. Genes of interest may be cloned in the multiple cloning site upstream of the sFv unit.

### a) Transfection of U937-1 cells with pHook-1 plasmid

U937-1 cells in late log phase are centrifuged at 340 x g for 5 minutes, washed once in PBS and resuspended in RPMI1640 medium to a concentration of 20 x 10⁶ cells/450 µl medium). Approximately 50 µg pHook-1 in 50 µl RPMI-1640 medium is added to the U937-1 cells and the solution is transferred to an electroporation cuvette. The cuvette is incubated on ice for 5 minutes before it is placed in the cuvette chamber. Electroporation is performed at 1000µF, ∞ Ω and 300 V. The cuvette is incubated on ice for 10 minutes and the contents are transferred to 50 ml RPMI-1640 containing 10% fetal calf serum, 2 mM L-glutamine and antibiotic (preincubated at 37°C). The transfected cells are incubated at 37°C and 5% CO₂.

### b) Preparation of [(5-oxo-2-phenyloxazol-4-ylidenemethyl)amino]acetic acid

To a stirred solution of 4-ethoxymethylene-2-phenyl-2-phenyl-2-oxazolin-5-one (4 mmol) in acetone (20 ml) under a nitrogen atmosphere is added a solution generated by stirring glycine (4 mmol) in sodium bicarbonate solution (20 ml) for 15 minutes. The mixture is stirred for 18 hours, washed with dichloromethane (2 x 15 ml) and acidified with 1N hydrochloric acid. The crystals which form upon stirring are collected and thoroughly washed with dichloromethane to obtain pure product.

### c) Preparation of [(5-oxo-2-phenyloxazol-4-ylidenemethyl)amino]acetyl-DSPE

The product from (b) (0.56 mmol) is dissolved in dimethylformamide (5 ml). N-methylmorpholine (1.68 mmol) is added, followed by DSPE (0.84 mmol) and benzotriazol-1-yloxy-tris(dimethylamino)phosphonium hexafluorophosphate (0.73 mmol). The mixture is stirred for 16 hours, whereafter dimethylformamide is evaporated under reduced pressure. The residue is taken up in ethyl acetate, the resulting solution is evaporated to dryness, and the residue is purified by flash chromatography on silica using chloroform/methanol (8:2) to give white crystals of the title compound (yield 70%).

### d) Preparation of gas microbubbles carrying the hapten phOx

Gas microbubbles encapsulated with DSPS "doped" with phOx-DSPE from (c) are prepared according to the method of Example 11(b).

### e) Separation of positively transfected cells

phOx-carrying microbubbles from (d) are added to proliferating transfected U937-1 cells in a centrifuge tube in a ratio of 10:1 and the tube is maintained at 37°C and placed on a roller mixer for 30 minutes. The tube is then centrifuged for 5 minutes at 200 x g. Cells bound to the microbubbles float to the top and are collected. The microbubbles are disrupted with a gentle overpressure and the transfected cells are used for further analyses. To verify the success of separation of transfected cells from non-transfected cells, single cells may be sorted in a flow cytometer and the genetic material of the cells analysed by the polymerase chain reaction.

### Example 17

### Synthesis of NH₂-Dab[PEG₃₄₀₀(N-α-acetyl-Cys)]-Lys(Hds)-Lys-Lys(Hds)-Glu-OH (where Dab = diaminobutyric acid and Hds = 2-n-hexadecylstearic acid), a thiol-containing PEGylated lipopeptide suitable for preparation of microbubbles for cell separation

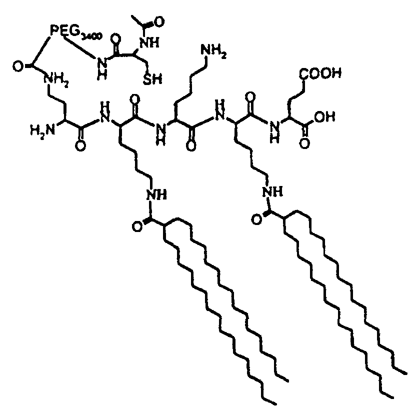

The peptide component of the above lipopeptide was synthesised on an ABI 433A automatic peptide synthesiser starting with Fmoc-Glu(OtBu)-Wang resin on a 0.2 mmol scale. Fmoc-Lys(Dde)-OH (1 mmol) was coupled using pre-activation with HATU. In a similar manner the amino acid derivatives in the order Fmoc-Lys(Boc)-OH, Fmoc-Lys(Dde)-OH and Boc-Dab(Fmoc)-OH were assembled automatically on the solid support. The peptide-resin was then transferred to a manual nitrogen bubbler and Fmoc-PEG₃₄₀₀-NHS (2 g, ca. 0.5 mmol) was coupled through the side chain of the Dab residue. Following removal of the resin-bound Fmoc group from the PEG spacer component with 20% piperidine in dimethylformamide, Fmoc-Cys(Trt) was coupled using HATU activation. Once again an Fmoc deprotection cycle was employed to liberate the Cys amino function, which was immediately capped with acetic anhydride. The Dde protecting groups were then cleaved in 2% hydrazine/dimethylformamide solution prior to coupling with 2-n-hexadecylstearic acid. Simultaneous removal of the PEGylated lipopeptide from the resin and side chain protecting groups was carried out in TFA containing 5% water and 5% triisopropylsilane for 2 hours, giving a crude product yield of 550 mg. Product characterization was carried out using MALDI mass spectrometry: expected M+H', multiple peaks from 4500-5200, found M+H⁺, 4000-5300.

### Example 18

### Isolation of haematopoietic progenitor cells from murine bone marrow with gas microbubbles encapsulated with hydrogenated egg phosphatidylserine

Bone marrow cells from femur/tibia of NMRI mice were obtained by flushing with MEM alpha culture medium. A cocktail of cell lineage specific antibodies directed against: CD2, CD8a, CD4, Mac-1, B220, Gr-1 and TER-119 (rat IgG isotype, PharMingen, San Diego, Ca) was added and the cells were incubated on ice for 30 minutes. Cells tagged with antibodies were removed with Dynabeads coated with sheep anti-rat IgG antibodies. The remaining lineage negative fraction was incubated for 30 minutes with hydrogenated egg phosphatidylserine-(HEPS-)encapsulated microbubbles "doped" with thiolated lipopepteide made as described in Example 5(a); the microbubbles were made as described in Example 5(b) using 4.0 mg HEPS instead of DSPS and using 0.7 mg lipopeptide. The cells fractionated by flotation constituted 2.44% of the lineage negative population and were assayed for their content of granulocyte/macrophage colony forming cells (GM-CFC) by culturing in agar dishes, 1000 cells per dish, in the presence of appropriate growth factors (SCF, IL-1, IL-3 and IL-6) After culture for 7 days in an incubator (with reduced oxygen partial pressure as a result of introduction of nitrogen into the air) the colonies were counted with an inverted microscope (Zeiss). More than every second cell was found to be a colony forming cell (530 colonies out of 1000 cells cultured).

### Example 19

### Preparation of gas microbubbles carrying nitrilotriacetic acid chelate binding centres

### a) Synthesis of N-[2-{Bis-carboxymethylamino}-6-(3-carboxypropionylamino)hexanoic acid]-1,2-distearoyl-sn-glycero-3-phosphoethanolamine

To the compound 1,2-distearoyl-sn-glycero-3-phosphoethanolamine-n-succinyl (Avanti Polar Lipids Inc., USA; 100 mg, 0.12 mmol) in dimethylformamide (5 ml) is added diisopropylethylamine (63 µl, 0.36 mmol) followed by a solution of *N,N,N',N'*-tetramethyl (succinimido)uronium tetrafluoroborate (TSTU) (45 mg, 0.15 mmol) in dimethylformamide (1 ml). The mixture is stirred for 2 hours, whereafter a solution of 2-(bis-carboxymethylamino)-6-(3-carboxypropionylamino)hexanoic acid (synthesised according to the procedure reported by Hochuli, E.; Dobeli, H.; Schacher, A.; Journal of Chromatography ***411*,** p.177 [1987]; 31.47 mg, 0.12 mmol) in dimethylformamide (1 ml) is added. The mixture is then stirred for 24 hours at ambient temperature, when monitoring by TLC shows the reaction to be complete. After evaporation of solvent the residue is purified by flash chromatography to afford pure white *title compound* (yield 65%).

### b) Preparation of gas microbubbles encapsulated with DSPC and chelate

To a mixture of 4.5 mg DSPC and 0.5 mg chelate from (a) is added 5% propylene glycol/glycerol in water (1 ml). The dispersion is heated to not more than 80°C for 5 minutes, then cooled to ambient temperature. The dispersion (0.8 ml) is transferred to a vial (1 ml) and the head space is flushed with perfluorobutane. The vial is shaken in a cap mixer for 45 seconds, whereafter the sample is put on a roller table. After centrifugation the infranatant is exchanged with water and the washing is repeated.

### c) Incorporation of metal cation into chelate-containing gas microbubbles

The chelate-containing microbubbles from (b) are further modified to allow the chelate to coordinate Ni²⁺ ions. The microbubbles are washed with 1 mM aqueous sodium hydroxide followed by 1% aqueous Ni(SO₄).6H₂ O, resulting in species coordinating Ni²⁺, thereby forming gas microbubbles comprising bound Ni²⁺.

## Claims

1. A process for the separation of target material from a liquid sample which comprises coupling the target to targetable encapsulated gas microbubbles, allowing the microbubbles and coupled target to float to the surface of the sample to form a floating microbubble/target layer, separating said layer from the sample, and either removing the microbubbles from the target or recovering target-free sample material.

2. A process as claimed in claim 1 wherein the gas microbubbles are encapsulated by a coalescence-resistant surface membrane, a filmogenic protein, a polymer material, a lipid material, a non-polymeric and non-polymerisable wall-forming material or a surfactant.

3. A process as claimed in claim 1 wherein the gas microbubbles are encapsulated by one or more phospholipids and/or lipopeptides.

4. A process as claimed in claim 3 wherein the gas microbubbles are encapsulated by membranes comprising at least two complementary lipopeptides.

5. A process as claimed in any of the preceding claims wherein the gas microbubbles bear a net overall charge.

6. A process as claimed in any of the preceding claims wherein the gas microbubbles comprise a perfluorocarbon or a sulphur fluoride.

7. A process as claimed in claim 6 wherein the gas microbubbles comprise sulphur hexafluoride, perfluoropropane or perfluorobutane.

8. A process as claimed in any of the preceding claims wherein the target material is selected from metals and metal ions, polymers, lipids, carbohydrates, blood components, proteins, glycoproteins, peptides, glycopeptides, hormones, immobilised combinatorial library components, cells, modified cells, cell fragments, cell organelles, DNA, RNA, phages, enzymes, ribosomes, toxins, bacteria, modified bacteria, viruses and modified viruses.

9. A process as claimed in claim 8 wherein the target material comprises hematopoietic cells or antigen presenting cells.

10. A process as claimed in claim 9 wherein said hematopoietic cells are selected from lymphocytes, granulocytes, monocytes, macrophages, reticulocytes, erythrocytes, megakaryocytes and platelets.

11. A process as claimed in claim 9 wherein said antigen presenting cells are selected from langerhans cells, endothelial cells, epithelial cells, trophoblasts and neural cells.

12. A process as claimed in claim 9 wherein the target material comprises hematopoietic progenitor cells and/or stem cells and the sample comprises a bone marrow/blood suspension or a cell culture containing hematopoietic progenitor cells and/or stem cells.

13. A process as claimed in claim 8 wherein the target material comprises cancer cells.

14. A process as claimed in claim 13 wherein said cancer cells are epithelial tumour cells.

15. A process as claimed in claim 8 wherein the target material comprises transfected cells or virus-infected cells.

16. A process as claimed in any of the preceding claims wherein the encapsulated gas microbubbles are rendered targetable by being coupled to an affinity ligand or vector either directly or through a linking group.

17. A process as claimed in claim 16 wherein said affinity ligand or vector is a monoclonal antibody.

18. A process as claimed in claim 16 wherein said affinity ligand or vector is a peptide or a secondary antibody having affinity for a primary antibody which has specificity for the target material.

19. A process as claimed in any of claims 1 to 15 wherein the encapsulated gas microbubbles are rendered targetable by attachment of a chelating agent or by the presence of one or more functional groups reactive with a complementary functional group in the target material.

20. A process as claimed in any of the preceding claims wherein the microbubbles are removed from the target by bursting.

21. A process as claimed in claim 20 wherein the microbubbles are burst by transient application of an overpressure or underpressure, by ultrasonication or by pH change.

22. A diagnostic method for the detection of a disease marker component in a liquid sample which comprises admixing said sample with encapsulated gas microbubbles capable of targeting said disease marker component, allowing said microbubbles and any coupled disease marker component to float to the surface of the sample to form a floating microbubble layer, and analysing said layer for the presence of said disease marker component.

23. Apparatus for use in the separation of components of a liquid sample by flotation, said apparatus comprising two chambers interconnected such that a microbubble-containing sample may be drawn into one chamber and microbubble/target component complexes may be allowed to float and then transferred into the other chamber.

24. Apparatus as claimed in claim 23 wherein said chambers comprise syringe barrels.

25. Apparatus for use in a continuous flow separation of components of a sample by flotation in accordance with the method of claim 1, said apparatus comprising feed means adapted to supply a continuous flow of targeted encapsulated gas microbubble-containing sample to a separation vessel having a downwardly converging side or sides, said vessel being equipped at its bottom with means for withdrawing sample liquid and unbound sample components and at its top with means permitting the overflow of sample liquid and microbubble/target component complexes.

## Patentansprüche

1. Verfahren zum Abtrennen eines Zielmaterials von einer flüssigen Probe, umfassend das Kuppeln des Ziels an zielfähige eingekapselte Gasmikrobläschen, Aufschwimmenlassen der Mikrobläschen und gekuppeltes Ziel zur Oberfläche der Probe zur Bildung einer schwimmenden Mikrobläschen/Zielschicht, Abtrennen der Schicht von der Probe, und entweder Entfernen der Mikrobläschen von dem Ziel oder Rückgewinnen von zielfreiem Probenmaterial.

2. Verfahren nach Anspruch 1, wobei die Gasmikrobläschen eingekapselt sind durch eine koaleszenzbeständige Oberflächenmembran, ein filmbildendes Material, ein Polymermaterial, ein Lipidmaterial, ein nicht polymeres und nicht polymerisierbares, wandbildendes Material oder ein Tensid.

3. Verfahren nach Anspruch 1, wobei die Gasmikrobläschen durch ein oder mehrere Phospholipide und/oder Lipopeptide eingekapselt sind.

4. Verfahren nach Anspruch 3, wobei die Gasmikrobläschen durch Membranen eingekapselt sind, umfassend mindestens zwei komplementäre Lipopeptide.

5. Verfahren nach mindestens einem der vorangehenden Ansprüche, wobei die Gasmikrobläschen eine Nettogesamtladung tragen.

6. Verfahren nach mindestens einem der vorangehenden Ansprüche, wobei die Gasmikrobläschen einen Perfluorkohlenstoff oder ein Schwefelfluorid umfassen.

7. Verfahren nach Anspruch 6, wobei die Gasmikrobläschen Schwefelhexafluorid, Perfluorpropan oder Perfluorbutan umfassen.

8. Verfahren nach mindestens einem der vorangehenden Ansprüche, wobei das Zielmaterial gewählt ist aus Metallen und Metallionen, Polymeren, Lipiden, Kohlenstoffhydraten, Blutkomponenten, Proteinen, Glycoproteinen, Peptiden, Glycopeptiden, Hormonen, immobilisierten kombinatorischen Bibliothekskomponenten, Zellen, modifizierten Zellen, Zellfragmenten, Zellorganellen, DNA, RNA, Phagen, Enzymen, Ribosomen, Toxinen, Bakterien, modifizierten Bakterien, Viren und modifizierten Viren.

9. Verfahren nach Anspruch 8, wobei das Zielmaterial blutbildende Zellen oder Antigen vorsehende Zellen umfasst.

10. Verfahren nach Anspruch 9, wobei die blutbildenden Zellen gewählt sind aus Lymphozyten, Granulozyten, Monozyten, Macrophagen, Retikulozyten, Erythrozyten, Megakaryozyten und Blutplättchen.

11. Verfahren nach Anspruch 9, wobei die Antigen vorsehenden Zellen gewählt sind aus Langerhans-Zellen, endothelialen Zellen, epithelialen Zellen, Trophoblasten und Nervenzellen.

12. Verfahren nach Anspruch 9, wobei das Zielmaterial blutbildende Progenitorzellen und/oder Stammzellen umfasst, und die Probe eine Knochenmark/Blutsuspension oder eine Zellkultur, enthaltend blutbildende Progenitorzellen und/oder Stammzellen umfasst.

13. Verfahren nach Anspruch 8, wobei das Zielmaterial Krebszellen umfasst.

14. Verfahren nach Anspruch 13, wobei die Krebszellen epitheliale Tumorzellen sind.

15. Verfahren nach Anspruch 8, wobei das Zielmaterial transfektierte Zellen oder Virus-infizierte Zellen umfasst.

16. Verfahren nach mindestens einem der vorangehenden Ansprüche, wobei die eingekapselten Gasmikrobläschen zielfähig gemacht werden, indem sie entweder direkt oder über eine Verbindungsgruppe an einen Affinitätsliganden oder - vektor gekuppelt werden.

17. Verfahren nach Anspruch 16, wobei der Affinitätsligand oder -vektor ein monoclonaler Antikörper ist.

18. Verfahren nach Anspruch 16, wobei der Affinitätsligand oder -vektor ein Peptid oder ein sekundärer Antikörper ist, der für einen primären Antikörper, welcher für das Zielmaterial Spezifizität besitzt, Affinität aufweist.

19. Verfahren nach mindestens einem der Ansprüche 1 bis 15, wobei die eingekapselten Gasmikrobläschen zielfähig gemacht werden durch Anbringung eines Komplexierungsmittels oder durch das Vorliegen einer oder mehrerer funktionellen Gruppen, welche mit einer komplementären funktionellen Gruppe in dem Zielmaterial reaktiv sind.

20. Verfahren nach mindestens einem der vorangehenden Ansprüche, wobei die Mikrobläschen von dem Ziel durch Bersten entfernt werden.

21. Verfahren nach Anspruch 20, wobei die Mikrobläschen durch vorübergehende Anwendung eines Überdrucks oder Unterdrucks, durch Ultraschall oder durch pH-Änderung geborsten werden.

22. Diagnostizierverfahren zur Detektion einer Krankheitsmarkerkomponente in einer flüssigen Probe, umfassend das Vermischen der Probe mit eingekapselten Gasmikrobläschen, welche fähig sind, die Krankheitsmarkerkomponente zu targetieren, Aufschwimmenlassen der Mikrobläschen und irgendeiner gekuppelten Krankheitsmarkerkomponente zur Oberfläche der Probe zur Bildung einer schwimmenden Mikrobläschenschicht, und Analysieren der Schicht auf das Vorhandensein der Krankheitsmarkerkomponente.

23. Vorrichtung zur Verwednung bei der Abtrennung von Komponenten einer flüssigen Probe durch Flotation, wobei die Vorrichtung zwei Kammern umfasst, die so miteinander verbunden sind, dass eine Mikrobläschen enthaltende Probe in eine Kammer eingezogen werden kann, und Mikrobläschen/Zielmaterial-Komlplexe aufschwimmen können und dann in die andere Kammer überführt werden können.

24. Vorrichtung nach Anspruch 23, wobei die Kammern Spritzengehäuse umfassen.

25. Vorrichtung zur Verwendung in einer kontinuierlichen Strömungstrennung von Komponenten einer Probe durch Flotation gemäß dem Verfahren nach Anspruch 1, wobei die Vorrichtung Einspeisemittel umfasst, adaptiert, um eine kontinuierliche Strömung einer targetierte eingekapselte Gasmikrobläschen enthaltende Probe einem Trennungsbehälter, der eine nach unten konvergierende Seite oder Seiten besitzt, zuzuführen, wobei der Behälter an seinem Boden mit Mitteln zum Abziehen von Probenflüssigkeit und ungebundener Probenkomponenten, und an seinem oberen Teil mit Mitteln, welche den Überlauf von Probenflüssigkeit und Mikrobläschen/Zielkomponente-Komplexen ermöglichen, ausgerüstet ist.

## Revendications

1. Procédé pour séparer un matériau cible d'un échantillon liquide, qui comprend le fait de coupler la cible à des microbulles de gaz encapsulées et aptes à être ciblées, de permettre aux microbulles et à la cible couplée de flotter sur la surface de l'échantillon pour former une couche flottante microbulles cible, de séparer ladite couche de l'échantillon, et soit d'éliminer les microbulles de la cible, soit de récupérer le matériau échantillon sans cible.

2. Procédé selon la revendication 1, dans lequel les microbulles de gaz sont encapsulées par une membrane de surface résistant à la coalescence, une protéine filmogène, un matériau polymère, un matériau lipidique, un matériau formant paroi, non polymère et non polymérisable, ou un tensioactif.

3. Procédé selon la revendication 1, dans lequel les microbulles de gaz sont encapsulées par un ou plusieurs phospholipides et/ou lipopeptides.

4. Procédé selon la revendication 3, dans lequel les microbulles de gaz sont encapsulées par des membranes comprenant au moins deux lipopeptides complémentaires.

5. Procédé selon l'une quelconque des revendications précédentes, dans lequel les microbulles de gaz portent une charge globale nette.

6. Procédé selon l'une quelconque des revendications précédentes, dans lequel les microbulles de gaz comprennent un perfluorocarbure ou un fluorure de soufre.

7. Procédé selon la revendication 6, dans lequel les microbulles de gaz comprennent de l'hexafluorure de soufre, du perfluoropropane ou du perfluorobutane.

8. Procédé selon l'une quelconque des revendications précédentes, dans lequel le matériau cible est choisi parmi les métaux et les ions métalliques, les polymères, les lipides, les glucides, les composants du sang, les protéines, les glycoprotéines, les peptides, les glycopeptides, les hormones, les composants immobilisés d'une pharmacothèque, les cellules, les cellules modifiées, les fragments cellulaires, les organelles cellulaires, l'ADN, l'ARN, les phages, les enzymes, les ribosomes, les toxines, les bactéries, les bactéries modifiées, les virus et les virus modifiés.

9. Procédé selon la revendication 8, dans lequel le matériau cible comprend des cellules hématopoïétiques ou des cellules présentant un antigène.

10. Procédé selon la revendication 9, dans lequel lesdites cellules hématopoïétiques sont choisies parmi les lymphocytes, les granulocytes, les monocytes, les macrophages, les réticulocytes, les érythrocytes, les mégacaryocytes et les plaquettes.

11. Procédé selon la revendication 9, dans lequel lesdites cellules présentant un antigène sont choisies parmi les cellules de Langerhans, les cellules endothéliales, les cellules épithéliales, les trophoblastes et les cellules neurales.

12. Procédé selon la revendication 9, dans lequel le matériau cible comprend des cellules progénitrices hématopoïétiques et/ou des cellules souches, et l'échantillon comprend une suspension de moelle osseuse/sang ou une culture cellulaire contenant des cellules progénitrices hématopoïétiques et/ou des cellules souches.

13. Procédé selon la revendication 8, dans lequel le matériau cible comprend des cellules cancéreuses.

14. Procédé selon la revendication 13, dans lequel lesdites cellules cancéreuses sont des cellules tumorales épithéliales.

15. Procédé selon la revendication 8, dans lequel le matériau cible comprend des cellules transfectées ou des cellules infectées par un virus.

16. Procédé selon l'une quelconque des revendications précédentes, dans lequel les microbulles de gaz encapsulées sont rendues aptes à être ciblées par couplage à un ligand d'affinité ou à un vecteur, directement ou par l'intermédiaire d'un groupe de liaison.

17. Procédé selon la revendication 16, dans lequel ledit ligand d'affinité ou ledit vecteur est un anticorps monoclonal.

18. Procédé selon la revendication 16, dans lequel ledit ligand d'affinité ou ledit vecteur est un peptide ou un anticorps secondaire ayant une affinité pour un anticorps primaire qui présente une spécificité pour le matériau cible.

19. Procédé selon l'une quelconque des revendications 1 à 15, dans lequel les microbulles de gaz encapsulées sont rendues aptes à être ciblées par fixation d'un agent chélatant, ou par la présence d'un ou plusieurs groupes fonctionnels pouvant réagir avec un groupe fonctionnel complémentaire dans le matériau cible.

20. Procédé selon l'une quelconque des revendications précédentes, dans lequel les microbulles sont enlevées de la cible par éclatement.

21. Procédé selon la revendication 20, dans lequel les microbulles sont éclatées par application transitoire d'une surpression ou d'une dépression, par application d'ultrasons ou par une variation du pH.

22. Procédé diagnostique pour détecter un composant marqueur de maladie dans un échantillon liquide, qui comprend le fait de mélanger ledit échantillon avec des microbulles de gaz encapsulées capables de cibler ledit composant marqueur de maladie, de permettre auxdites microbulles et à tout composant marqueur de maladie couplé, de flotter à la surface de l'échantillon pour former une couche de microbulles flottantes, et d'analyser ladite couche pour déterminer la présence dudit composant marqueur de maladie.

23. Appareil pour utilisation dans la séparation de composants d'un échantillon liquide par flottation; ledit appareil comprenant deux compartiments interconnectés, de façon qu'un échantillon contenant des microbulles puisse être amené dans un compartiment, et que des complexes microbulles/composant cible puissent flotter, et ensuite être transférés dans l'autre compartiment.

24. Appareil selon la revendication 23, dans lequel lesdits compartiments comprennent des tubes de seringue.

25. Appareil pour utilisation dans une séparation à flux continu de composants d'un échantillon par flottation, conformément au procédé selon la revendication 1, ledit appareil comprenant des moyens d'alimentation adaptés pour amener un flux continu d'un échantillon contenant des microbulles de gaz encapsulées ciblées vers un récipient de séparation comportant un ou des côtés convergeant vers le bas, ledit récipient étant, à sa partie inférieure, équipé avec des moyens pour soutirer le liquide échantillon et les composants non liés de l'échantillon, et, à sa partie supérieure, avec des moyens permettant le débordement de liquide échantillon et de complexes microbulles/composant cible.
